# EUROPÄISCHE PATENTANMELDUNG

(11) **EP 3 871 732 A1**
(43) Veröffentlichungstag der Anmeldung: **01.09.2021**
(21) Anmeldenummer: 20159671.5
(22) Anmeldetag: 27.02.2020
(51) Int. Cl.: A61M 37/00

(54) **ANORDNUNG ZUM ÜBERTRAGEN EINER ANTRIEBSKRAFT FÜR EINEN ANTRIEBSSTRANG EINER VORRICHTUNG ZUM LOKALEN AUFSTECHEN EINER MENSCHLICHEN ODER EINER TIERISCHEN HAUT, ANTRIEBSSTRANG UND HAUTSTECHVORRICHTUNG**

(71) Anmelder: MT.DERM GmbH, 14167 Berlin (DE)
(72) Erfinder: FRISTER, Dirk, 13053 Berlin (DE); LISEC, Walter, 13053 Berlin (DE)
(74) Vertreter: Bittner, Thomas L.

(57) **Zusammenfassung**

Anordnung und Vorrichtung zum lokalen Aufstechen einer Haut, mit: einer antriebsseitigen Kopplung (1), die eingerichtet ist, mit einer Antriebseinrichtung (7) über eine antriebsseitige Verbindung verbunden zu werden und von der Antriebseinrichtung (7) eine Antriebskraft zu empfangen; einer abtriebsseitigen Kopplung (3), die eingerichtet ist, mit einer Hautstecheinrichtung (8) über eine abtriebsseitige Verbindung verbunden zu werden und die Antriebskraft an die Hautstecheinrichtung (8) abzugeben; einem Zwischenkoppelbauteil (2), welches zum Übertragen der Antriebskraft mit der antriebseitigen Kopplung (1) und der abtriebsseitigen Kopplung (3) verbunden ist; und einer Einstelleinrichtung (4), die dem Zwischenkoppelbauteil (2) zugeordnet ist, eine Eingangsgröße, welche für eine Vorrichtung zum lokalen Aufstechen einer menschlichen oder einer tierischen Haut im Betrieb einen Betriebsparameter anzeigt, zu empfangen und einen am Zwischenkoppelbauteil (2) bereitgestellten Kopplungsfaktor, welcher die Übertragung der Antriebskraft von der antriebsseitigen Kopplung mittels des Zwischenkoppelbauteils (2) auf die abtriebsseitige Kopplung (3) charakterisiert, in Abhängigkeit von der Eingangsgröße einzustellen.

## Beschreibung

Die Erfindung betrifft eine Anordnung zum Übertragen einer Antriebskraft für einen Antriebsstrang einer Vorrichtung zum lokalen Aufstechen einer menschlichen oder einer tierischen Haut, einen Antriebsstrang sowie eine Hautstechvorrichtung.

### Hintergrund

Vorrichtungen zum lokalen Aufstechen einer menschlichen oder einer tierischen Haut verfügen regelmäßig über eine Antriebseinrichtung, von der eine Antriebskraft bereitgestellt wird. Die Antriebskraft kann als repetierende Antriebskraft mit einer Wiederholfrequenz bereitgestellt werden, zum Beispiel mit einem elektromotorischen Antrieb. Alternativ kann vorgesehen sein, eine manuelle Antriebskraft bereitzustellen. Die von der Antriebseinrichtung zur Verfügung gestellte Antriebskraft wird über einen Antriebsstrang auf eine Stecheinrichtung übertragen, die mit einer Stechnadel gebildet ist, die zum lokalen Aufstechen der Haut üblicherweise durch eine vorderseitige Gehäuseöffnung aus- und eingefahren wird. Hierbei führt die Stechnadel eine lineare Bewegung (Hub) aus.

In dem Dokument DE 10 2014 012 896 A1 ist für eine Hautstechvorrichtung ein Adapter mit einer Amplitudenuntersetzung offenbart. Im Antriebsstrang sind miteinander gekoppelte Masse-Federsysteme vorgesehen, die dazu dienen, die antriebsseitig mit einem Hub eingebrachte repetitive Bewegung in einem kleineren Hub auf der Abtriebsseite zur Hautstecheinrichtung hin umzusetzen. Für ein antriebsseitiges Federelement kann ein Kopplungsfaktor produktionsseitig fest oder mittels eines Verstellmechanismus vom Anwender variabel eingestellt werden, um unterschiedliche Amplitudenreduktionen zwischen der Antriebs- und der Abtriebsseite zu realisieren.

### Zusammenfassung

Aufgabe der Erfindung ist es, eine Anordnung zum Übertragen einer Antriebskraft für einen Antriebsstrang einer Vorrichtung zum lokalen Aufstechen einer menschlichen oder einer tierischen Haut, einen Antriebsstrang sowie eine Hautstechvorrichtung anzugeben, die eine für verschiedene Anwendungssituationen eine flexible Anpassbarkeit der Kraftübertragung zwischen Antriebs- und Abtriebsseite ermöglichen.

Zur Lösung sind eine Anordnung zum Übertragen einer Antriebskraft für einen Antriebsstrang einer Vorrichtung zum lokalen Aufstechen einer menschlichen oder einer tierischen Haut nach dem unabhängigen Anspruch 1 sowie ein Antriebsstrang und eine Hautstechvorrichtung nach den nebengeordneten Ansprüchen 10 und 11 vorgesehen. Ausgestaltungen sind Gegenstand von abhängigen Unteransprüchen.

Nach einem Aspekt ist eine Anordnung zum Übertragen einer Antriebskraft für einen Antriebsstrang einer Vorrichtung zum lokalen Aufstechen einer menschlichen oder einer tierischen Haut mit folgenden Merkmalen geschaffen: eine antriebsseitige Kopplung, die eingerichtet ist, mit einer Antriebseinrichtung über eine antriebsseitige Verbindung verbunden zu werden und hierüber von der Antriebseinrichtung eine Antriebskraft zu empfangen; eine abtriebsseitige Kopplung, die eingerichtet ist, mit einer Haustecheinrichtung über eine abtriebsseitige Verbindung verbunden zu werden und hierüber die Antriebskraft an die Hautstecheinrichtung abzugeben; ein Zwischenkoppelbauteil, welches zum Übertragen der Antriebskraft mit der antriebseitigen Kopplung und der abtriebsseitigen Kopplung verbunden ist; und eine Einstelleinrichtung, die dem Zwischenkoppelbauteil zugeordnet und eingerichtet ist, eine Eingangsgröße, welche für eine Vorrichtung zum lokalen Aufstechen einer menschlichen oder einer tierischen Haut im Betrieb einen Betriebsparameter anzeigt, zu empfangen und einen am Zwischenbauteil bereitgestellten Kopplungsfaktor, welcher die Übertragung der Antriebskraft von der antriebsseitigen Kopplung mittels des Zwischenkoppelbauteils auf die abtriebsseitige Kopplung charakterisiert, in Abhängigkeit von der Eingangsgröße einzustellen.

Nach weiteren Aspekten ist ein Antriebsstrang für eine Vorrichtung zum lokalen Aufstechen einer menschlichen oder einer tierischen Haut mit der Anordnung sowie eine Hautstechvorrichtung geschaffen.

Mithilfe der vorgeschlagenen Technologie ist es ermöglicht, die antriebsseitig bereitgestellte Antriebskraft flexibel und regelbar auf der Abtriebsseite bereitzustellen und an die Hautstecheinrichtung weiterzugeben. Hierbei kann die abtriebsseitig abgegebene Antriebskraft kleiner oder gleich der auf der Antriebsseite von der Antriebseinrichtung empfangenen Antriebskraft eingestellt werden. Es ist so eine automatisierte Einstellmöglichkeit für den Kopplungsfaktor und somit für die Übertragung der Antriebskraft bereitgestellt, welche eine betriebsabhängige Regelung ermöglicht.

Die über das Zwischenkoppelbauteil übertragene Antriebskraft kann für aufeinanderfolgende Hubbewegungen gleich oder verschieden sein. Es kann vorgesehen sein, die abtriebsseitig auf die Hautstecheinrichtung eingeleitete Antriebskraft während einer Hubbewegung zum Aufstechen der Haut im Wesentlichen konstant einzustellen, was mithilfe der Einstelleinrichtung ermöglicht ist. Auch über die Zeit einer Hubbewegung nicht konstante oder sich verändernde Antriebskräfte können mithilfe eines entsprechenden Betriebs der Einstelleinrichtung abtriebsseitig bereitgestellt werden.

Der Einstelleinrichtung kann eine Steuereinrichtung zugeordnet sein, mit der unterschiedliche zeitliche Verläufe der Eingangsgröße, welche für die Vorrichtung zum lokalen Aufstechen der menschlichen oder der tierischen Haut im Betrieb den Betriebsparameter anzeigt, in zugeordnete Steuerprofile umgesetzt werden, also einen zeitabhängigen Verlauf von Steuersignalen, die von der Einstelleinrichtung zum Einstellen des Kopplungsfaktors am Zwischenbauteil angewendet werden.

Es kann eine Messeinrichtung vorgesehen sein, die mit der Einstelleinrichtung verbunden und eingerichtet ist, Messsignale für den Betriebsparameter zu erfassen und hieraus die an die Einstelleinrichtung weiterzugebende Eingangsgröße zu bestimmen. Die Messeinrichtung kann für eine kontaktbehaftete oder eine kontaktlose Messung eingerichtet sein, um die Messsignale zu erfassen. Die Messeinrichtung kann in die Vorrichtung zum lokalen Aufstechen der menschlichen oder der tierischen Haut integriert sein, beispielsweise in ein gemeinsames Vorrichtungsgehäuse. Die Messeinrichtung kann ein oder mehrere Sensoren aufweisen, die eingerichtet sind, gleiche oder verschiedene physikalische Messgrößen zu detektieren.

Die Messeinrichtung kann eingerichtet sein, Messsignale für mindestens einen der folgenden Betriebsparameter zu erfassen: Weg einer Bewegung eines Bauteils oder eines Bauteilabschnitts, Drehzahl eines Motorantriebs der Antriebseinrichtung und an dem Motorantrieb anliegende Last. Der Weg der Bewegung eines Bauteils oder eines Bauteilabschnitts kann eine charakteristische Messgröße für die Hubbewegung anzeigen. Als Reaktion auf die unterschiedlichen Messsignale kann die Einstelleinrichtung den Kopplungsfaktor, ausgehend von einem momentanen Wert, erhöhen oder vermindern, um so die abtriebsseitig an die Hautstecheinrichtung abgegebene Antriebskraft hinsichtlich ihrer Größe zu verändern. Bei dieser oder anderen Ausführungsformen kann ein Stechwerkzeug der Hautstecheinrichtung mit einer Einzelnadel oder einem mehrere Nadelspitzen aufweisendem Nadelmodul ausgebildet sein. Auch eine Gruppe von Einzelnadeln kann gemeinsam das Stechwerkzeug bilden.

Mit dem Zwischenkoppelbauteil kann mindestens eine der folgenden Koppelarten bereitgestellt sein: mechanische Kopplung, pneumatische Kopplung, magnetische Kopplung und elektrische Kopplung.

Das Zwischenkoppelbauteil kann einen elastisch verformbaren Koppelkörper aufweisen, und der Kopplungsfaktor kann einstellbar sein, indem ein der Verformung entgegenwirkender Verformungswiderstand mittels der Einstelleinrichtung für den elastisch verformbaren Koppelkörper einstellbar ist. Der an den Zwischenkoppelbauteil bereitgestellte Kopplungsfaktor hängt bei dieser Ausgestaltung von der elastischen Verformung des Koppelkörpers ab. Eine im Ausmaß größere elastische Verformung des Koppelkörpers (geringerer Verformungswiderstand) führt dazu, dass ein größerer Teil der antriebsseitig bereitgestellten Antriebskraft von dem Koppelkörper aufgenommen und so nicht abtriebsseitig an die Hautstecheinrichtung abgegeben wird. Umgekehrt bewirkt eine geringere elastische Verformung des Koppelkörpers, dass die antriebsseitig bereitgestellte Antriebskraft in größerem Umfang an die Abtriebsseite abgegeben wird.

Das Zwischenkoppelbauteil kann ein Reservoir mit einem viskosen Material aufweisen, und die Einstelleinrichtung kann eingerichtet sein, zum Einstellen des am Zwischenbauteil bereitgestellten Verformungswiderstands eine Viskosität des viskosen Materials einzustellen. Als viskoses Material können Flüssigkeit zum Einsatz kommen. Auch eine Kombination unterschiedlicher viskoser Materialien kann vorgesehen sein.

Alternativ kann in dem Reservoir ein Elastomermaterial zur Anwendung kommen, für die mittels der Einstelleinrichtung die Materialelastizität einstellbar ist.

Das viskose Material kann mit einem magnetorheologischen Material gebildet sein. Magnetorheologischen Materialien verändern ihre Festigkeit (Verformbarkeit) in Abhängigkeit eines angelegten Magnetfelds. Soll im Betrieb einer Hautstechvorrichtung beispielsweise die abtriebsseitig abgegebene Antriebskraft erhöht werden, kann mittels der Einstelleinrichtung ein an das magnetorheologische Material angelegtes Magnetfeld vergrößert werden.

Das Zwischenkoppelbauteil kann eine Magnetfeder aufweisen, und die Einstelleinrichtung kann eingerichtet sein, zum Einstellen des am Zwischenkoppelbauteil bereitgestellten Verformungswiderstands eine magnetische Federkraft an der Magnetfeder einzustellen.

Das Zwischenkoppelbauteil kann eine mechanische Federeinrichtung aufweisen, und die Einstelleinrichtung kann eingerichtet sein, zum Einstellen des am Zwischenkoppelbauteil bereitgestellten Verformungswiderstands eine mechanische Federkraft an der mechanische Federeinrichtung einzustellen. Die mechanische Federeinrichtung kann beispielsweise eine oder mehrere Spiralfedern aufweisen.

In Verbindung mit dem Antriebsstrang sowie der Hautstechvorrichtung können die vorangehend im Zusammenhang mit der Anordnung für den Antriebsstrang erläuterten Ausgestaltungen entsprechend vorgesehen sein.

Bei der Hautstechvorrichtung kann vorgesehen sein, dass der Antriebsstrang antriebsseitig mit einer eine repetierende oder sich wiederholende Antriebskraft bereitstellenden Antriebseinrichtung verbunden ist. Beispielsweise kann ein elektromotorischer Antrieb vorgesehen sein. Hierbei kann von einem Motor eine drehende Antriebsbewegung bereitgestellt werden, die mithilfe einer Wandlungseinrichtung in eine wiederholende Vor- und Rückbewegung (lineare) Bewegung gewandelt wird. Die Antriebseinrichtung kann eingerichtet sein, die repetierende Antriebskraft mit einer Wiederholfrequenz im Bereich von etwa 5 Hz bis etwa 200 Hz bereitzustellen, alternativ im Bereich von etwa 20 Hz bis etwa 50 Hz.

Bei der Hautstechvorrichtung kann die Einstelleinrichtung eingerichtet sein, als Eingangsgröße einen Betriebsparameter zu empfangen, welcher die repetierende Antriebskraft oder eine hierdurch beeinflusste Messgröße anzeigt, um den Kopplungsfaktor in Abhängigkeit hiervon einzustellen.

### Beschreibung von Ausführungsbeispielen

Im Folgenden werden weitere Ausführungsbeispiele unter Bezugnahme auf Figuren einer Zeichnung näher erläutert. Hierbei zeigen:
- Fig. 1: eine schematische Darstellung einer Anordnung für einen Antriebsstrang einer Vorrichtung zum lokalen Aufstechen einer menschlichen oder einer tierischen Haut;
- Fig. 2: eine schematische Darstellung eines Antriebsstrangs einer Vorrichtung zum lokalen Aufstechen einer menschlichen oder einer tierischen Haut mit der Anordnung aus Fig. 1;
- Fig. 3: eine schematische Darstellung einer Anordnung für ein Zwischenkoppelbauteil mit einem Koppelkörper mit magnetisch beeinflussbarer elastischer Verformbarkeit;
- Fig. 4: eine schematische Darstellung einer Anordnung für ein Zwischenkoppelbauteil mit einer Magnetfeder und
- Fig. 5: eine schematische Darstellung einer Anordnung für ein Zwischenkoppelbauteil mit einer magnetischen Kopplung.

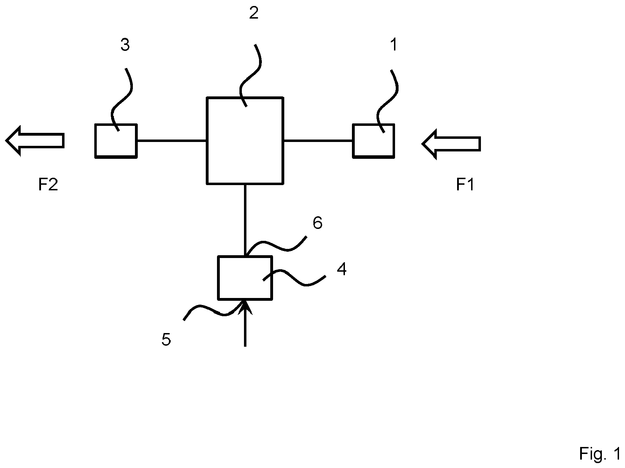

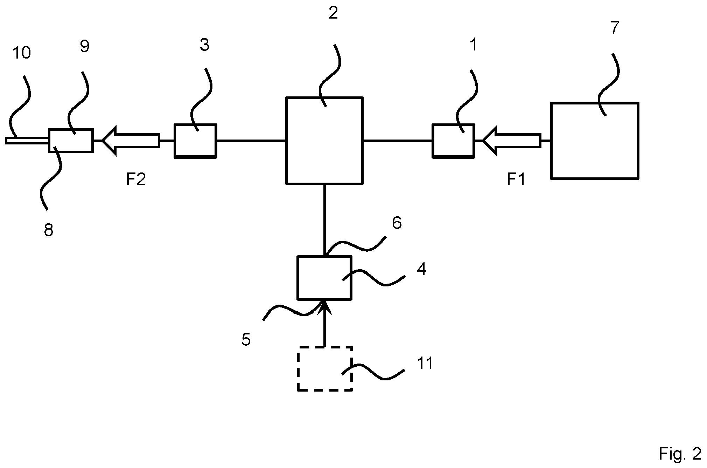

Fig. 1 zeigt eine schematische Darstellung einer Anordnung für einen Antriebsstrang einer Vorrichtung zum lokalen Aufstechen einer menschlichen oder einer tierischen Haut. Über eine antriebsseitige Kopplung 1 kann eine von einer Antriebseinrichtung (vgl. Fig. 2) bereitgestellte Antriebskraft empfangen werden. Die empfangene Antriebskraft wird über ein Zwischenkoppelbauteil 2 auf eine abtriebsseitige Kopplung 3 übertragen, welche eingerichtet ist, die abtriebsseitige Antriebskraft an eine Hautstecheinrichtung (vgl. Fig. 2 abzugeben). Dem Zwischenkoppelbauteil 2 ist eine Einstelleinrichtung 4 zugeordnet. Die Einstelleinrichtung 4 empfängt über einen Eingang 5 eine Eingangsgröße und ist eingerichtet, über einen Ausgang 6 eine Einstellgröße bereitzustellen, mit der an dem Zwischenkoppelbauteil 3 ein betriebsabhängiger Kopplungsfaktor eingestellt wird, der charakterisiert, in welchem Umfang oder Ausmaß die antriebsseitig bereitgestellte Antriebskraft mittels des Zwischenkoppelbauteils 2 auf die Abtriebsseite übertragen wird. Es können Kopplungsfaktoren eingestellt werden, bei denen die antriebsseitig empfangene Antriebskraft in vollem oder in geringerem Umfang auf die Abtriebsseite übertragen wird. Auf diese Weise wird eine variable Bereitstellung der abtriebsseitigen Antriebskraft in Abhängigkeit von der Einstellgröße von der Einstelleinrichtung 4 ermöglicht.

Fig. 2 zeigt eine schematische Darstellung eines Antriebsstrangs für eine Vorrichtung zum lokalen Aufstechen einer menschlichen oder einer tierischen Haut mit der Anordnung aus Fig. 1. Antriebsseitig koppelt die Anordnung aus Fig. 1 an eine Antriebseinrichtung 7. Abtriebsseitig wird die dort bereitgestellte Antriebskraft von der abtriebsseitigen Kopplung 3 an eine Hautstecheinrichtung 8 übertragen, bei der in einer Nadelaufnahme 9 ein Stechwerkzeug 10 angeordnet ist, welches aufgrund der Einleitung der abtriebsseitigen Antriebskraft im Betrieb wiederholt (repetierend) vor- und zurückbewegt wird. Zum Ausbilden einer Hautstechvorrichtung können die in Fig. 2 dargestellten Elemente oder Komponenten in einem ein- oder mehrstückigen Gehäuse aufgenommen werden.

Die Einstelleinrichtung 4 kann mit einer Messeinrichtung 11 verbunden sein, um hierüber die Eingangsgröße zu empfangen. Die Messeinrichtung 11 erfasst im Betrieb eine oder mehrere Messgrößen für Betriebsparameter. Hierzu kann beispielsweise eine Wegstrecke der Hubbewegung der Hautstecheinrichtung 8 gehören. Alternativ oder ergänzend kann eine Last an der Antriebseinrichtung 7 detektiert werden. Eine oder mehrere Messgrößen können zeitabhängig mittels der Messeinrichtung 11 erfasst werden, um hieraus Steuersignale zum Einstellen des Kopplungsfaktors abzuleiten.

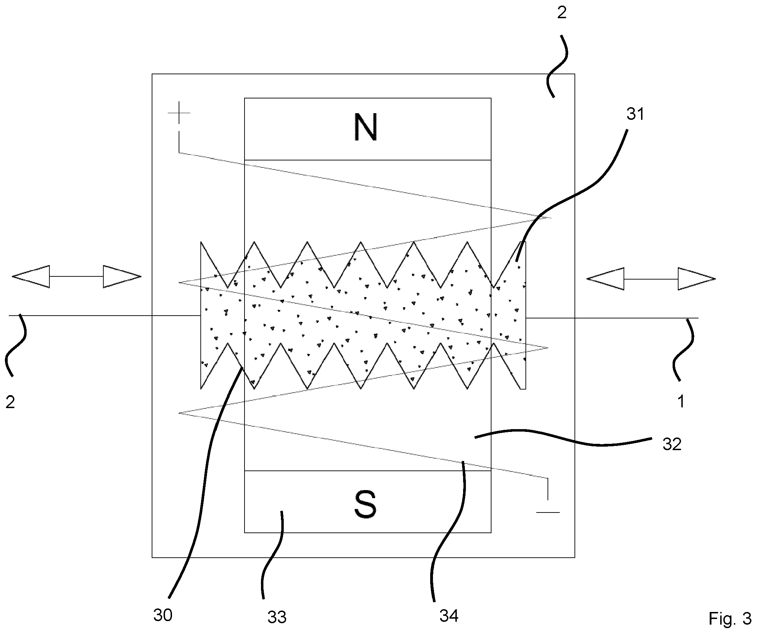

In den Fig. 3 bis 5 sind unterschiedliche Mechanismen für die Übertragung der antriebsseitig empfangenen Antriebskraft auf die Abtriebsseite in dem Zwischenkoppelbauteil 2 gezeigt. Bei der Ausführungsform in Fig. 3 ist ein Reservoir 30 mit einem viskosen Material 31 vorgesehen, bei dem es sich im gezeigten Beispiel um ein magnetorheologisches Material handelt. Das magnetorheologische Material ist elastisch komprimierbar (verformbar), wenn über die antriebsseitige Kopplung 1 die Antriebskraft eingekoppelt wird, um diese über die abtriebsseitige Kopplung 3 abzugeben. Ein Magnetfeld 32, welches beispielhaft mittels einer Kombination von Permanentmagnet 33 und stromdurchflossener Spule 34 gebildet ist, wird mittels der Einstelleinrichtung 4 eingestellt, um dem Komprimieren des magnetorheologischen Materials entgegengesetzten Widerstand betriebsabhängig einzustellen. Zum Beispiel können magnetorheologische Elastomere zum Einsatz kommen, in der Regel aus einer Elastomermatrix und darin dispergierten magnetisch aktiven Partikeln bestehen. Bei diesen Elastomeren können die viskoelastischen oder dynamisch-mechanischen Eigenschaften mittels Anlegen eines äußeren Magnetfeldes schnell und reversibel verändert werden.

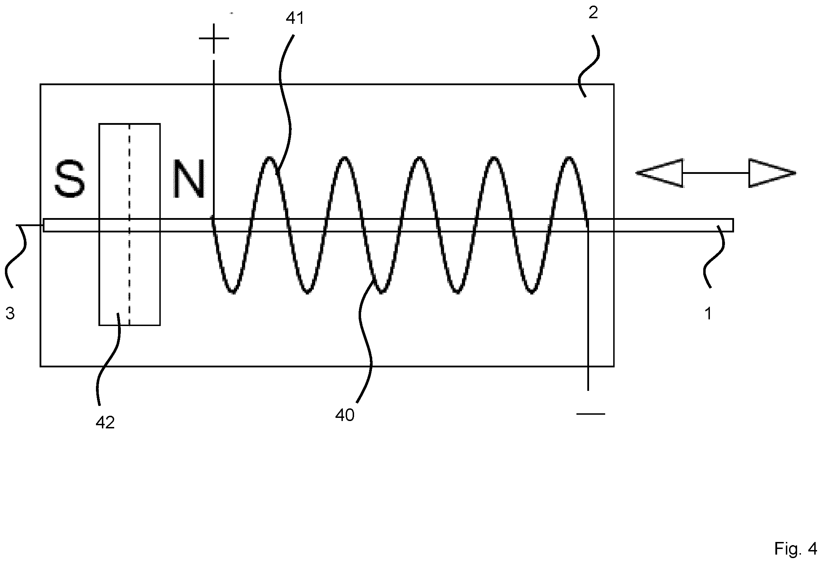

In Fig. 4 ist eine Ausführungsform dargestellt, die für das Zwischenkoppelbauteil 2 eine einstellbare Magnetfeder 40 vorsieht. Ein feststehender Elektromagnet 41 ist mit einer elektrischen stromdurchflossenen Spule gebildet. Gegenüberliegend ist ein Permanentmagnet 42 entlang des Antriebsstrangs vorgesehen, welcher mit der abtriebsseitigen Kopplung 3 verbunden ist, um abtriebsseitig die Antriebskraft abzugeben. Mittels Steuerung der elektrischen Beaufschlagung der Spule kann der Kopplungsfaktor betriebsabhängig eingestellt werden.

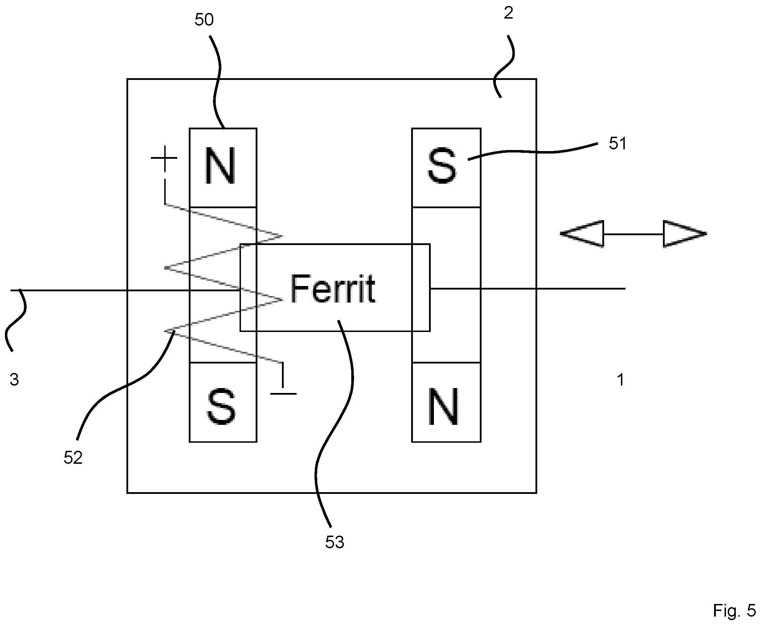

Bei der Ausgestaltung in Fig. 5 ist eine Anordnung mit feststehenden Permanentmagneten 50, 51 sowie einer stromdurchflossenen Spule 52 vorgesehen. In dem bereitgestellten Magnetfeld ist entlang des Antriebsstrangs ein Ferrit-Bauteil 53 angeordnet. Mittels Steuerung der elektrischen Beaufschlagung der Spule 52 kann der Kopplungsfaktor eingestellt werden.

Die in der vorstehenden Beschreibung, den Ansprüchen sowie der Zeichnung offenbarten Merkmale können sowohl einzeln als auch in beliebiger Kombination für die Verwirklichung der verschiedenen Ausführungen von Bedeutung sein.

## Patentansprüche

1. Anordnung zum Übertragen einer Antriebskraft für einen Antriebsstrang einer Vorrichtung zum lokalen Aufstechen einer menschlichen oder einer tierischen Haut, mit:
- einer antriebsseitigen Kopplung (1), die eingerichtet ist, mit einer Antriebseinrichtung (7) über eine antriebsseitige Verbindung verbunden zu werden und hierüber von der Antriebseinrichtung (7) eine Antriebskraft zu empfangen;
- einer abtriebsseitigen Kopplung (3), die eingerichtet ist, mit einer Hautstecheinrichtung (8) über eine abtriebsseitige Verbindung verbunden zu werden und hierüber die Antriebskraft an die Hautstecheinrichtung (8) abzugeben;
- einem Zwischenkoppelbauteil (2), welches zum Übertragen der Antriebskraft mit der antriebseitigen Kopplung (1) und der abtriebsseitigen Kopplung (3) verbunden ist; und
- einer Einstelleinrichtung (4), die dem Zwischenkoppelbauteil (2) zugeordnet und eingerichtet ist, eine Eingangsgröße, welche für eine Vorrichtung zum lokalen Aufstechen einer menschlichen oder einer tierischen Haut im Betrieb einen Betriebsparameter anzeigt, zu empfangen und einen am Zwischenkoppelbauteil (2) bereitgestellten Kopplungsfaktor, welcher die Übertragung der Antriebskraft von der antriebsseitigen Kopplung mittels des Zwischenkoppelbauteils (2) auf die abtriebsseitige Kopplung (3) charakterisiert, in Abhängigkeit von der Eingangsgröße einzustellen.

2. Anordnung nach Anspruch 1, **gekennzeichnet durch** eine Messeinrichtung (11), die mit der Einstelleinrichtung (4) verbunden und eingerichtet ist, Messsignale für den Betriebsparameter zu erfassen und hieraus die an die Einstelleinrichtung (4) weiterzugebende Eingangsgröße zu bestimmen.

3. Anordnung nach Anspruch 2, **dadurch gekennzeichnet, dass** die Messeinrichtung (11) eingerichtet ist, Messsignale für mindestens einen der folgenden Betriebsparameter zu erfassen: Weg einer Bewegung eines Bauteils oder eines Bauteilabschnitts, Drehzahl eines Motorantriebs der Antriebseinrichtung (7) und an dem Motorantrieb anliegende Last.

4. Anordnung nach mindestens einem der vorangehenden Ansprüche, **dadurch gekennzeichnet, dass** mit dem Zwischenkoppelbauteil (2) mindestens eine der folgenden Koppelarten bereitgestellt ist: mechanische Kopplung, pneumatische Kopplung, magnetische Kopplung und elektrische Kopplung.

5. Anordnung nach mindestens einem der vorangehenden Ansprüche, **dadurch gekennzeichnet, dass** das Zwischenkoppelbauteil (2) einen elastisch verformbaren Koppelkörper aufweist und der Kopplungsfaktor einstellbar ist, indem ein der Verformung entgegenwirkender Verformungswiderstand mittels der Einstelleinrichtung für den elastisch verformbaren Koppelkörper einstellbar ist.

6. Anordnung nach Anspruch 5, **dadurch gekennzeichnet, dass** das Zwischenkoppelbauteil (2) ein Reservoir (30) mit einem viskosen Material (31) aufweist und die Einstelleinrichtung (4) eingerichtet ist, zum Einstellen des am Zwischenkoppelbauteil (2) bereitgestellten Verformungswiderstands eine Viskosität des viskosen Materials (31) einzustellen.

7. Anordnung nach Anspruch 6, **dadurch gekennzeichnet, dass** das viskose Material mit einem magnetorheologischen Material (31) gebildet ist.

8. Anordnung nach mindestens einem der Ansprüche 5 bis 7, **dadurch gekennzeichnet, dass** das Zwischenkoppelbauteil (2) eine Magnetfeder aufweist und die Einstelleinrichtung (4) eingerichtet ist, zum Einstellen des am Zwischenkoppelbauteil (2) bereitgestellten Verformungswiderstand eine magnetische Federkraft an der Magnetfeder einzustellen.

9. Anordnung nach mindestens einem der Ansprüche 5 bis 8, **dadurch gekennzeichnet, dass** das Zwischenkoppelbauteil (2) eine mechanische Federeinrichtung aufweist und die Einstelleinrichtung (4) eingerichtet ist, zum Einstellen des am Zwischenkoppelbauteil (2) bereitgestellten Verformungswiderstandes eine mechanische Federkraft an der mechanische Federeinrichtung einzustellen.

10. Antriebsstrang für eine Vorrichtung zum lokalen Aufstechen einer menschlichen oder einer tierischen Haut, mit einer Anordnung nach mindestens einem der vorangehenden Ansprüche.

11. Hautstechvorrichtung zum lokalen Aufstechen einer menschlichen oder einer tierischen Haut, mit einem Antriebsstrang nach Anspruch 10.

12. Hautstechvorrichtung nach Anspruch 11, **dadurch gekennzeichnet, dass** der Antriebsstrang antriebsseitig mit einer eine repetierende Antriebskraft bereitstellenden Antriebseinrichtung (7) verbunden ist.

13. Hautstechvorrichtung nach Anspruch 12, **dadurch gekennzeichnet, dass** die Einstelleinrichtung (4) eingerichtet ist, als Eingangsgröße einen Betriebsparameter zu empfangen, welcher die repetierende Antriebskraft oder eine hierdurch beeinflusste Messgröße anzeigt, um den Kopplungsfaktor in Abhängigkeit hiervon einzustellen.
